# EUROPEAN PATENT APPLICATION

(11) **EP 3 178 401 A1**
(43) Date of publication of application: **14.06.2017**
(21) Application number: 16163995.0
(22) Date of filing: 06.04.2016
(51) Int. Cl.: A61B 8/00, A61B 8/08, A61B 8/14

(54) **ULTRASONIC DIAGNOSTIC APPARATUS AND METHOD FOR CONTROLLING THE SAME**

(30) Priority: 08.12.2015 KR 20150174129
(71) Applicant: Samsung Medison Co., Ltd., Hongcheon-gun, Gangwon-do, 25108 (KR)
(72) Inventor: PARK, Sung-Ah, Seoul (KR); YANG, Eun Ho, Seoul (KR)
(74) Representative: Land, Addick Adrianus Gosling

(57) **Abstract**

Disclosed is an ultrasonic diagnostic apparatus and method for controlling the same, which increases user convenience and shortens time required for the user to re-adjust a region of interest (ROI) by automatically creating an ROI to fit a changed object image if the user makes a mode change. The ultrasonic diagnostic apparatus includes a probe for irradiating an ultrasound signal to an object and receiving an echo ultrasound signal from the object; an image generator for creating an image in a first mode based on the echo ultrasound signal received by the probe and creating an image in a second mode related to the first mode; an image measurer for measuring the object image on the image in the first mode; a region of interest (ROI) generator for automatically creating an ROI in the second mode based on the measured object image as the first mode is changed to the second mode; and a display for displaying the image in the first mode, and displaying the image in the second mode with the created ROI as the mode change is made.

## Description

Embodiments of the present disclosure relate to an ultrasonic diagnostic apparatus and method for controlling the same, which changes a region of interest (ROI) depending on the position and size of an object.

An ultrasonic diagnostic apparatus is used for medical purposes, such as non-invasively acquiring images of layers of soft tissue or blood flows of an internal part of an object by irradiating ultrasound signals generated from transducers of a probe from the surface of the object toward a target part inside the object and receiving information of reflected ultrasound signals (echo ultrasound signals), observing the internal part of the object, detecting foreign materials, analyzing damage, etc.

Compared to other diagnostic imaging apparatuses, such as X-ray diagnostic apparatuses, X-ray Computerized Tomography (CT) scanners, Magnetic Resonance Imaging (MRI) apparatuses, nuclear medicine diagnostic apparatuses, etc., the ultrasonic diagnostic apparatus has many advantages that they are compact, inexpensive, able to display in real time, and safe because of no exposure to radiation, and is thus widely used with other kinds of diagnostic imaging apparatus.

The ultrasonic diagnostic apparatus selects an image, boundary or particular point of the object displayed on the ultrasound image to analyze a distance or volume of the object. With the conventional ultrasonic diagnostic apparatus, the user has to manually change a region of interest (ROI) each time the user makes a mode change on the ultrasound image.

Exemplary embodiments of the present disclosure are directed to an ultrasonic diagnostic apparatus and method for controlling the same, which increases user convenience and shortens time required for the user to re-adjust a region of interest (ROI) by automatically creating an ROI to fit a changed object image if the user makes a mode change.

According to an aspect of the present disclosure, a ultrasonic diagnostic apparatus includes a probe for irradiating an ultrasound signal to an object and receiving an echo ultrasound signal from the object; an image generator for creating an image in a first mode based on the echo ultrasound signal received by the probe and creating an image in a second mode related to the first mode; an image measurer for measuring the object image on the image in the first mode; a region of interest (ROI) generator for automatically creating an ROI in the second mode based on the measured object image as the first mode is changed to the second mode; and a display for displaying the image in the first mode, and displaying the image in the second mode with the created ROI as the mode change is made.

The image in the first mode may include an image created in B mode.

The second mode may include at least one of power Doppler mode, color Doppler mode, directional power Doppler mode, elasticity mode, and 3D mode.

The image generator may include an input unit for receiving a command to control at least one of the image measurer and the ROI generator.

The image generator may create the image in the second mode related to the image in the first mode under the control of the input unit.

The image measurer may measure a size or volume of the object image in the first mode under the control of the input unit.

The ROI generator may edit the ROI created in the second mode under the control of the input unit.

The ROI generator may create the ROI according to setting values input through the input unit based on the size or volume of the object measured by the image measurer.

The ROI generator may create the ROI according to a setting value of unmeasured length or breadth of the object input through the input unit if the image measurer measures one of the length and breadth of the object.

The ROI generator may modify the ROI to fit the image in the second mode, if the ROI goes beyond the image in the second mode.

The ROI generator may create an ROI in the second mode based on angular points of a measured area of the object, if the image measurer measures the object while rotating around the object.

The ROI generator may create an ROI in the second mode based on a point at which the measured object is bent, if the image measurer measures the object as having the form of a bending straight line in measuring the size of the object.

The ROI generator may create an ROI in the second mode based on a measured curvature of a curve, if the image measurer measures the object as having the form of the curve or a free curve in measuring the size of the object.

According to another aspect of the present disclosure, a method for controlling an ultrasonic diagnostic apparatus includes irradiating an ultrasound signal to an object and receiving an echo ultrasound signal from the object; creating an image according to a mode based on the received echo ultrasound signal; measuring the object image on the image in a first mode; creating a region of interest (ROI) in a second mode based on the measured object image as the first mode is changed to the second mode; and displaying the image in the first mode, and displaying the image in the second mode with the created ROI as the mode change is made.

The image in the first mode may include an image created in B mode.

The second mode may include at least one of power Doppler mode, color Doppler mode, directional power Doppler mode, elasticity mode, and 3D mode.

Measuring the object image on the image in a first mode may include measuring a size or volume of the object image on the image in the first mode.

The method may further include editing the created ROI under the control of a user.

Creating an ROI may include creating the ROI according to setting values based on the measured size or volume of the object.

Creating an ROI may include creating the ROI according to a setting value of unmeasured length or breadth, if one of length and breadth of the object is measured in measuring the size of the object.

Creating an ROI may include modifying the ROI to fit the image in the second mode, if the ROI goes beyond the image in the second mode.

Creating an ROI may include creating an ROI in the second mode based on angular points of a measured area of the object, if the object is measured while being rotated.

Creating an ROI may include creating an ROI in the second mode based on a point at which the measured object is bent, if the object is measured as having the form of a bending straight line in measuring the size of the object.

Creating an ROI may include creating an ROI in the second mode based on a measured curvature of a curve, if the object is measured as having the form of the curve or a free curve in measuring the size of the object.

### BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
FIG. 1 is an exterior view of an ultrasonic diagnostic apparatus, according to an embodiment of the present disclosure;
FIG. 2 is a control block diagram of an ultrasonic diagnostic apparatus, according to an embodiment of the present disclosure;
FIG. 3 is a table illustrating menus for measuring an object on an ultrasound image;
FIG. 4 is a flowchart illustrating a method for automatically creating a region of interest (ROI) when an ultrasonic diagnostic mode is changed, according to an embodiment of the present disclosure;
FIG. 5 shows an example of automatically editing and changing an ROI after a mode change is made;
FIG. 6 shows an example of editing and creating a three dimensional (3D) ROI;
FIG. 7 shows an example of automatically editing and creating an ROI in a case of adjusting a function of the present disclosure;
FIG. 8 shows an example of automatically creating an ROI by editing an old ROI created beyond an image screen; and
FIGS. 9 to 12 show examples of automatically editing and creating many different shapes of ROI.

### DETAILED DESCRIPTION OF EXEMPLARY EMBODIMENTS

Embodiments of an ultrasonic diagnostic apparatus and method for controlling the same will be described in detail with reference to accompanying drawings. Like reference numerals refer to like components throughout the drawings, and thus the related descriptions that overlap will be omitted.

The term "object" as herein used may include a person or animal, or a part of the person or animal. For example, the object may include an organ, such as the liver, heart, uterus, brain, breasts, abdomen, etc., or a vein as well as a mass. The term 'user' as herein used may be a doctor, a nurse, a medical technologist, a medical image expert, etc., or a technician who fixes medical equipment, but is not limited thereto.

The terms "ultrasound image" and "object image" as herein used may include an image of an object, which is obtained not only using ultrasounds, but also using an X-ray diagnostic apparatus, a Computerized Tomography (CT) scanner, a Magnetic Resonance Image (MRI) device, or a nuclear medicine diagnostic apparatus.

A diagnostic apparatus for which technologies of an ultrasonic diagnostic apparatus and method for controlling the same in accordance with embodiments of the present disclosure may be applied and used may be expanded to one of an X-ray scanning apparatus, an X-ray fluoroscopic apparatus, a CT scanner, an MRI, a positron emission tomography apparatus, and an ultrasonic diagnostic apparatus. Embodiments of the present disclosure take the ultrasonic diagnostic apparatus as an example, but are not limited thereto.

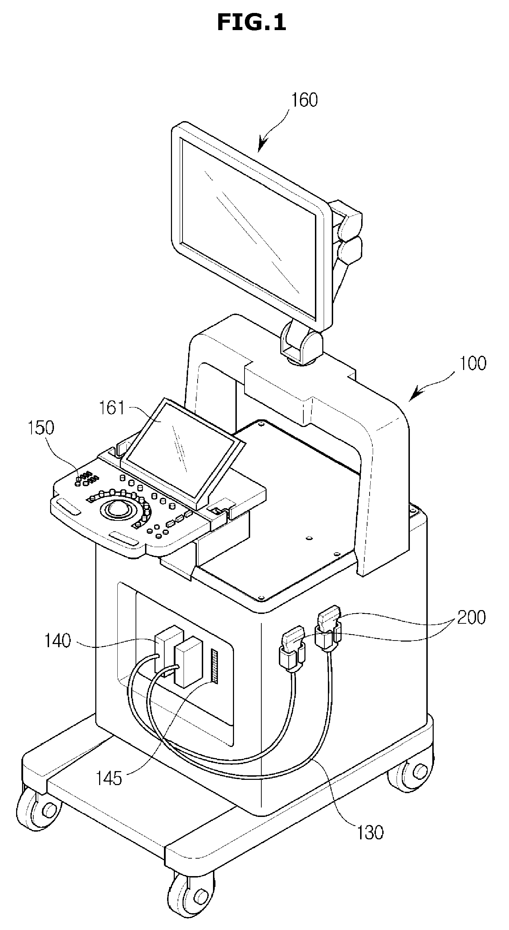

FIG. 1 is an exterior view of an ultrasonic diagnostic apparatus, according to an embodiment of the present disclosure.

Referring to FIG. 1, an ultrasonic diagnostic apparatus may include a main unit 100, and an input unit 150, a display 160, a sub display panel 161, and a probe 200, which are connected to the main unit 100.

On the bottom side of the main unit 100, there may be multiple casters (not shown) for mobility of the ultrasonic diagnostic apparatus. The multiple casters may fix the ultrasonic diagnostic apparatus to a particular location, or may move the ultrasonic diagnostic apparatus to a particular direction. Such an ultrasonic diagnostic apparatus may be referred to as a cart type ultrasonic diagnostic apparatus.

Alternatively, the ultrasonic diagnostic apparatus may be a portable ultrasonic diagnostic apparatus that may be carried for long distance. In this case, the portable ultrasonic diagnostic apparatus may not include any caster. For example, the portable ultrasonic diagnostic apparatus may be a PACS viewer, smart phone, laptop computer, personal digital assistant (PDA), tablet personal computer (PC), etc., but is not limited thereto.

The probe 200 may contact the surface of the body of an object, transmitting/receiving ultrasounds to/from the object. Specifically, the probe 200 serves to transmit ultrasounds to the inside of the object according to an ultrasound signal, which is an electronic signal provided from the main unit 100, collect echo ultrasounds reflected from a particular internal part of the object, and forward corresponding echo ultrasound signals to the main unit 100.

For this, the probe 200 may include transducers and a multiplexer (MUX) circuit. The transducers may include a plurality of elements for converting electric signals to ultrasounds through vibration or converting ultrasounds to electric signals. The plurality of elements may be arrayed on a face of the housing of the probe 200. Specifically, the plurality of transducers may be arrayed in a direction parallel to an opening formed on a face of the housing, such that ultrasounds may be transmitted/received through the opening.

The probe 200 may be connected to an end of a cable 130, the other end of which may be connected to a male connector 140. The male connector 140 connected to the other end of the cable 130 may be mechanically combined with a female connector 145 of the main unit 100.

In this way, a single probe 200 may be connected to the main unit 100, or similarly, a plurality of probes 200 may be connected to the single main unit 100. For the latter case, a plurality of male connectors may be mounted on the main unit 100. In FIG. 1, two probes 200 are connected to the single main unit 100.

The probe 200 may be connected to the main unit 100 wirelessly. In this case, the probe 200 may wirelessly transmit echo ultrasound signals corresponding to echo ultrasounds received from the object to the main unit 100.

The main unit 100 may send ultrasound signals to the probe 200, receive echo ultrasound signals from the probe 200, and create ultrasound images based on the received echo ultrasound signals.

The ultrasound image may be provided to the user through the display 160. The user may diagnose the object, e.g., a patient, while visually checking the ultrasound image of an internal part of the object through the display 160.

The display 160 may display various user interfaces (UIs) related to controlling the ultrasonic diagnostic apparatus. The user may check the UI provided through the display 160, and input a control command for the ultrasonic diagnostic apparatus or a component of the ultrasonic diagnostic apparatus through the input unit 150.

Furthermore, the display 160 may display an ultrasound image acquired in the process of ultrasonic diagnosis. The display 160 may be implemented with one of a Cathode Ray Tube (CRT), a Liquid Crystal Display (LCD), etc., and may provide not only 2D images but also 3D images.

By touching the display 160, the user may input a control command related to the ultrasonic diagnostic apparatus as well as input a touch command to set a region of interest (ROI) on the ultrasound image of the object, in which the user intends to perform examination and diagnosis.

Furthermore, the display 160 may display a gain compensation control to compensate a gain of the ultrasound image. The ultrasound image may vary in sharpness and brightness of image quality depending on depths or differences in lateral pixel values.

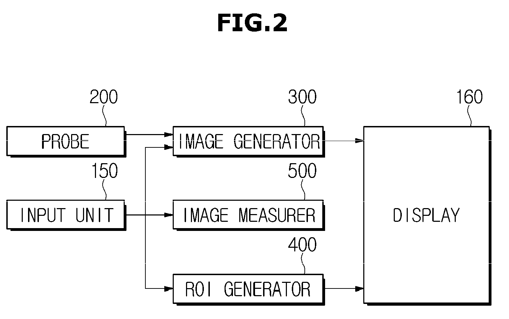

In accordance with an embodiment of the present disclosure, the display 160 may display an object image included in a part set by the user as the ROI on the ultrasound image of the object. Details of ROI settings and modifications will be described later in connection with FIG. 2.

The user may set an ROI by touching the display 160 displaying the ultrasound image.

For this, the display 160 may include a touch panel for receiving the touch input of the user, and the touch panel may be implemented with e.g., an LCD panel, a Light Emitting Diode (LED) panel, an Organic Light Emitting Diode (OLED) panel, or the like.

Similar to the display 160, the sub display panel 161 may display various UIs related to controlling the ultrasonic diagnostic apparatus, and the user may check the UI provided through the sub display panel 161, and input a control command for the ultrasonic diagnostic apparatus or a part of the ultrasonic diagnostic apparatus through the input unit 150 or a touch screen of the sub display panel 161.

Furthermore, the sub display panel 161 may display ultrasound images acquired in the process of ultrasonic diagnosis, and the user may input a control command related to the ultrasonic diagnostic apparatus or a command related to setting an ROI on the ultrasound image by touching the sub display panel 161.

For this, the sub display panel 161 may include a touch panel for receiving the touch input of the user, and the touch panel may be implemented with e.g., an LCD panel, an LED panel, an OLED panel, or the like.

The input unit 150 may receive a command related to an operation of the ultrasonic diagnostic apparatus 100. The user may input commands to start a diagnosis, select a part for diagnosis, select a diagnosis type, select a mode for a finally output ultrasound image, etc., through the input unit 150.

Furthermore, the user may input command related to selecting and modifying an ROI on an ultrasound image displayed on the display unit 160 or sub display panel 161 through the input unit 150, and re-adjust the changed ROI on the ultrasound image displayed on the display unit 160 or sub display panel 161. Controlling of an ROI will be described later in detail in connection with FIG. 2.

The input unit 150 may be located on the top of the main unit 100, as shown in FIG. 1. The input unit 150 may include at least one of switches, keys, a wheel, a joy stick, a trackball, and a knob, but is not limited thereto.

FIG. 2 is a control block diagram of an ultrasonic diagnostic apparatus, according to an embodiment of the present disclosure.

Referring to FIG. 2, an ultrasonic diagnostic apparatus in accordance with an embodiment of the present disclosure may include the input unit 150, the display 160, the probe 200, an image generator 300, an ROI generator 400, and an image measurer 500.

The probe 200 includes a plurality of transducer elements for mutually converting between ultrasounds and electric signals. The transducer elements transmit ultrasound signals to an object, and receive echo ultrasound signals reflected from the object to generate receive signals.

Specifically, the probe 200 may transmit focused ultrasound beams to the object along transmit scan lines by properly delaying input time of pulses input to the respective transducer elements. Echo ultrasound signals reflected from the object may be input to the respective transducer elements at different reception points in time, and each transducer element may output the input echo ultrasound signal.

A beamformer (not shown) focuses ultrasounds onto a particular point by controlling time to drive respective transducers of the probe 200 in transmitting the ultrasounds, and converts analog signals received from the probe 200 to digital signals. A received focus signal may be generated by receiving and focusing the digital signal taking into account the locations of the transducer elements and the focusing point.

The image generator 300 may create an ultrasound image by performing e.g., an envelope detection process to detect the amplitude of the ultrasound signal focused by the beamformer.

A method for creating an ultrasound image may vary with each mode. In other words, the image generator 300 may generate various images depending on diagnostic modes. There are different types of diagnostic modes for the ultrasonic diagnostic apparatus as herein used, such as B mode, M mode, Doppler mode, and color Doppler mode. Furthermore, even 2D mode and 3D mode may correspond to the diagnostic mode for the ultrasonic diagnostic apparatus.

Specifically, B mode is to display a cross-sectional image of an internal part of an object, representing parts having strong echoes and weak echoes in differences in brightness. In other words, the B mode refers to a mode to display a 2D ultrasound image by representing energy of signals reflected from the object in levels of brightness.

Doppler mode refers to a mode to display an image based on Doppler effect that causes the change in frequency of a sound output from a moving object. Such a mode using the Doppler effect may be further divided into Power Doppler Imaging (PDI) mode, color Doppler mode, and Directional Power Doppler Imaging (DPDI) mode.

Power Doppler mode, i.e., the PDI mode represents a level of a Doppler signal or the number of structures (e.g. the number of red blood cells in the blood) in an image that has no aliases because of being less sensitive to the incident angle, and has less image attenuation from noise. Furthermore, the power Doppler mode records reflected Doppler energy, and may thus be very sensitive even to detect narrow veins and slow blood streams.

The color Doppler mode provides a velocity image that represents the velocity of a Doppler signal in a 2D distribution. The image in the color Doppler mode may not only visualize blood flow in real time but also represent a wide range of blood flow patterns from high-speed blood flow in a wide vein to low-speed blood flow in a narrow vein.

The DPDI mode refers to a mode to display a directional image that represents directional information of a Doppler signal in the PDI mode in a 2D distribution. Accordingly, the DPDI mode has an advantage over the PDI mode in that it allows more accurate detection of information regarding blood flow.

An elasticity mode refers to a method for acquiring an image of an object using elastography. Elastography means analyzing a phenomenon that the harder a structure like a malignant mass, the lower the elasticity of the tissues and thus, the smaller a difference in metamorphosis of the tissues. Especially, it is used a lot in the field of diagnosing breast cancer or prostate cancer.

The 3D mode may refer to a mode to display an image indicating a geometric solid or space with x, y, z values representing depth, width, height, respectively, or to provide a 3D form to give a feeling of stereoscopy or display a series of images with the stereoscopic effect. For example, using the stereoscopic effect of the 3D mode, the user may display the shape of a face of a fetus to be shown to his/her parents.

In the present disclosure, diagnostic modes of the ultrasonic diagnostic apparatus are not limited to what is mentioned above but may include other various modes.

Turning back to FIG. 2, if a new image is created as the diagnosis mode is changed according to a control signal from the input unit 150, the ROI generator 400 may automatically create an ROI to fit the new image.

Specifically, the ultrasonic diagnostic apparatus may create a 2D ultrasound image for the object by means of the image generator 300. In this regard, the image measurer 500 may measure the size and volume of the object on the created image. After that, if the user makes a change of diagnosis mode through the input unit 150, the ROI generator 400 may automatically create an ROI on an image in the changed mode.

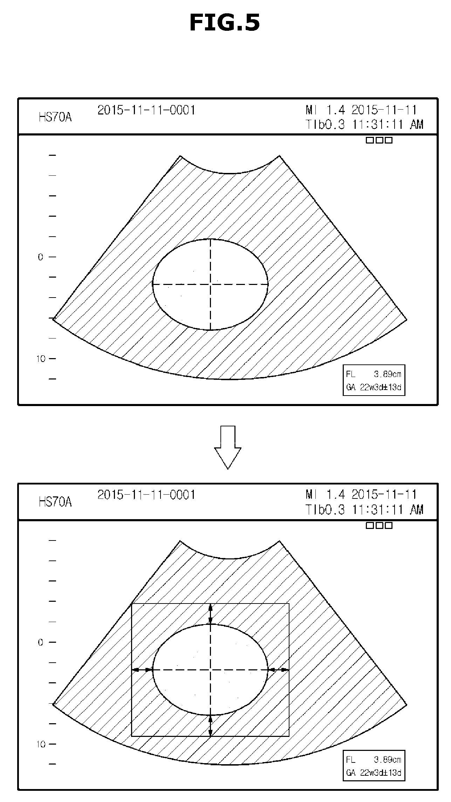

Meanwhile, the shape of the ROI created by the ROI generator 400 may vary according to the probe 200. That is, there may be various shapes of ROI, as will be described below. Accordingly, there may be various ways for the ROI generator 400 to automatically edit and modify an ROI, and various modifications of them will be described later in connection with FIGS. 5 to 11.

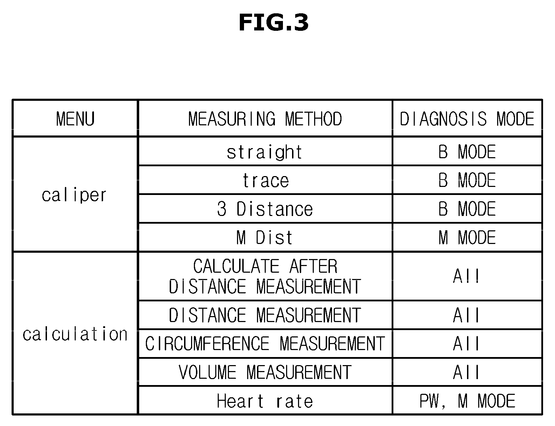

The image measurer 500 measures the object on the image created by the image generator 200. Specifically, the image measurer 500 may calculate at least one of the length, breadth, and volume of the object based on the size of the created image. How to measure the object image will be described later in connection with FIG. 3.

The user may determine a single mode or a plurality of modes through the input unit 150, and the image generator 300 may create an image in each mode.

The input unit 150 may also receive a command from the user to measure the object on the generated image.

For example, the input unit 150 may control the image generator 300 to create an image in the B mode, and to re-create the image as the diagnosis mode is changed from the B mode to the color Doppler mode.

The input unit 150 may receive and send a menu input to measure the object to the image measurer 500, and control the image measurer 500 to measure the object on the image in the B mode.

Furthermore, the input unit 150 may control the ROI generator 400. For example, the user may change e.g., the position and size of the ROI automatically created as the mode change is made, through the input unit 150.

In the meantime, the input unit 150 may be implemented with a key board, a trackball, a mouse, a touch panel, or the like, and if the display 160 is implemented with a touch screen, the touch screen may perform both functions of the display and the input unit, but is not limited thereto.

The display 160 may display various UIs related to controlling the ultrasonic diagnostic apparatus 100, display ultrasound images acquired in the process of ultrasonic diagnosis, and also provide not only 2D images but also 3D images. Description related to the display 160 that overlaps what is described above in connection with FIG. 1 will be omitted herein.

The ultrasonic diagnostic apparatus of the present disclosure may include other functions and features not shown in FIG. 2.

Specifically, there is a memory (not shown) for storing information regarding the created ultrasound images and ROI generation. Furthermore, the memory may separately store an images within an ROI.

Alternatively, the user may store the information regarding ROI generation and settings, and after that, the user may easily perform observation on the ROI on the ultrasound image based on the stored data.

The memory may include e.g., a high-speed random access memory (RAM), a magnetic disk, a static RAM, a dynamic RAM, a read only memory (ROM), etc., but is not limited thereto. Moreover, the memory may be detachable from the ultrasonic diagnostic apparatus. For example, the memory may include a compact flash (CF) card, a secure digital (SD) card, a smart media (SM) card, a multimedia card (MMC), or a memory stick, but is not limited thereto.

In addition, the memory may be separated from the ultrasonic diagnostic apparatus and may transmit or receive data to or from the ultrasonic diagnostic apparatus via cable or wirelessly.

The aforementioned image generator 300, ROI generator 400, image measurer 500, and memory may be incorporated in a single processor. Specifically, the processor may be implemented in an array of multiple logic gates, or in a combination of a universal microprocessor and a memory that stores a program executable in the microprocessor, but is not limited thereto.

FIG. 3 is a table illustrating menus for measuring an object on an ultrasound image.

Referring to FIG. 3, the table includes menus, measuring methods and diagnosis modes available for the measuring methods included in each menu.

Menus refer to methods for analyzing the size or volume of an object, including Caliper and Calculation menus.

The image measurer 500 may analyze the size of the object based on the selected menu. An analysis method may be set in advance, and the user may selectively set an analysis method through the input unit 150.

The Caliper menu is a method for analyzing an object, including distance analysis, circumference and area analysis, and volume analysis. For the distance analysis, a straight method for analyzing a straight line distance and a trace method for analyzing the contour of a curve may be included.

The Caliper menu also refers to a method for measuring the volume of an object through a 3 Distance method. The 3 Distance method is for analyzing typical x, y, and z values to mathematically calculate a volume. Meanwhile, the volume analysis method of the Caliper menu may be realized not only by the 3 Distance method but also by MOD and Ellipse methods, and may include other various volume analysis methods.

In addition, the Caliper menu may include various measuring methods, such as M Dist, 2 line, spline, etc. In other words, in embodiments of the present disclosure, there may be various ways for the image measurer 500 to measure an object without limitations.

Measuring methods in the Caliper menu, i.e., Straight, Trace, and 3 Distance may all be used in the B mode, an ultrasonic diagnosis mode. However, the M Dist measuring method in the Caliper menu may only be used in M mode.

Specifically, the image measurer 500 may measure the object image created in the B mode through Straight, Trace or 3 Distance method, and the ROI generator 400 may generate an ROI based on the measurements of the object image.

The image measurer 500 may measure the object image according to the Calculation menu in addition to the Caliper menu.

The Calculation menu may include a measuring method to calculate the object after distance measurement. For example, a distance, circumference, and volume of the object is measured using a measurement item (UI) called Mass. The Calculation menu may also be used in the majority of diagnosis modes, but in which case some modes have some limitations. The user may perform measurement of distance or volume for various measurement items of the Calculation menu through the input unit 150.

Table shown in FIG. 3 is only way of example, and there may be other various menus and measuring methods in the present disclosure.

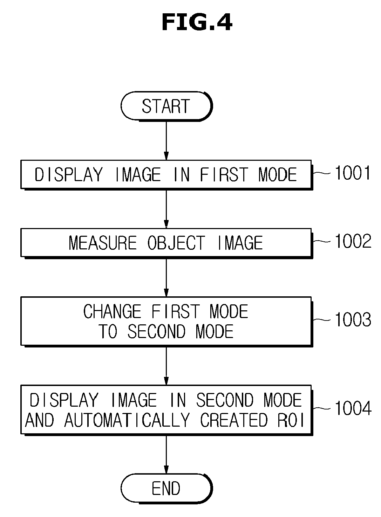

FIG. 4 is a flowchart illustrating a method for automatically creating an ROI as an ultrasonic diagnostic mode is changed, according to an embodiment of the present disclosure.

The image generator 300 displays an image in a first mode on the display 160, in operation 1001.

Specifically, the user uses the probe 200 to irradiate ultrasound signals to an object, and the probe 200 then receives echo ultrasound signals. The probe 200 then sends the echo ultrasound signals to a beamformer (not shown), which in turn, converts the received signals in an analog form to digital signals.

The image generator 300 creates an ultrasound image under a mode selected in advance by the user, based on ultrasound signals focused by the beamformer. The mode selected in advance by the user refers to the first mode.

For example, if the user sets the first mode to B mode, the image generator 300 represents parts having strong and weak echoes in differences of brightness, and creates a 2D black-and-white image, i.e., an image in B mode to represent such differences. The image generator 300 may send the image created in the B mode to the display 160, and thus the user may check the created ultrasound image through the display 160.

The image measurer 500 measures an object on the image in the first mode, in operation 1002.

As described above in connection with FIG. 3, the image measurer 500 measures the object image through a measuring method according to a menu selected by the user through the input unit 150.

For example, if the user selects the Trace method of the Caliper menu as a measuring method in the B mode, the image measurer 500 may measure the size of the object image by tracing pixel values in an area where difference in brightness is distinct.

In another example, if the user sets a measuring method to the Distance measuring method of the Calculation menu, the image measurer 500 may measure the length and breadth of the 2D object image on the image in the B mode.

The user may then change the diagnosis mode from the first mode to a second mode, in operation 1003. For example, the user may make a mode change in order to more clearly figure out the object or to examine the object under another mode.

For example, assume that the user sets the first mode to the B mode and the ultrasonic diagnostic apparatus displays a 2D black-and-white image in the B mode. The object displayed on the ultrasound image may be a mass.

The user may change the ultrasonic diagnosis mode from the B mode to the color Doppler mode to more clearly figure out whether the object is a mass and examine the mass together with a blood stream flowing around the mass. Here, the first mode corresponds to the B mode, and the second mode changed from the first mode corresponds to the color Doppler mode.

Specifically, the input unit 150 receives an input signal of the user and accordingly controls the image generator 300. The image generator 300 recreates an image based on the changed diagnosis mode and sends the recreated image to the display 160.

In the meantime, the ROI generator 400 automatically creates an ROI by tracing the object image on the image modified based on the second mode, in operation 1004.

Specifically, the ROI generator 400 creates an ROI based on the object image measured in the first mode. The ROI generator 400 may generate the ROI in various forms, and the ROI is created based on the previously measured size or volume of the object.

For example, if the image measurer 500 measures the object in a 2D size having length and breadth, the ROI generator 400 may create an ROI in the form of a rectangular box by extending the measurements of the length and breadth to some extent.

The ROI generator 500 then sends the created ROI to the display 160, which in turn, displays the image in the 2D mode and the created ROI together.

This eliminates the need for the user to newly create an ROI or change an ROI position through the input unit 150 each time the user makes a mode change, thereby increasing convenience of use of the ultrasonic diagnostic apparatus.

In the meantime, the user may edit the created ROI through the input unit 150. The user may also set an ROI generation function to be automatically turned on or off through the input unit 150. For example, the ultrasonic diagnostic apparatus 100 of the present disclosure may allow the user to determine whether to automatically change and create an ROI each time a mode change is made.

FIG. 5 shows an example of automatically creating an ROI in a changed mode.

Referring to FIG. 5, the upper view briefly depicts a 2D black-and-white image (first image) in the B mode. An arrow between the upper and lower views means that a mode change is made. The lower view briefly depicts an image (second image) in the changed second mode with a created ROI.

Specifically, the user may set or input a control signal to create an image related to the B mode to the image generator 300 through the input unit 150 in advance. Based on the control signal, the image generator 300 creates an ultrasound image for an object, i.e., a 2D black-and-white image and display it through the display 160.

In the first image, the form of a circle briefly represents a mass.

The image measurer 500 measures the mass image on the first image. The dotted cross within the mass image is an image depicted to explain the Straight measuring method. In other words, the image measurer 500 may measure the length and breadth of the object image (i.e., the mass image in this example).

After that, if the user makes a change of diagnosis mode, the image generator 300 recreates an image based on the newly selected diagnosis mode. Along with this, the ROI generator 400 creates an ROI as big as a predetermined size, i.e., a default size based on the measured object image.

Specifically, in the second image, the ROI generator 400 creates an ROI in the form of a rectangular box by extending the dotted lines of the measured length and breadth of the object to a certain extent. Bidirectional arrows depicted within the rectangular box correspond to the certain extent.

The ROI generator 400 and the image generator 300 may send the created image and ROI to the display 160, and the display 160 may display the received image and ROI.

As such, the present disclosure may save trouble of the user having to manually move the ROI to fit an object image displayed in a changed mode each time a change in diagnosis mode is made, thereby increasing the user convenience.

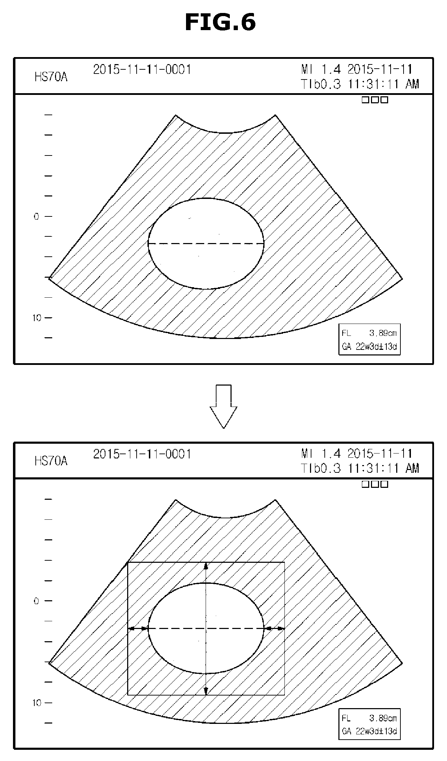

FIG. 6 shows another example of automatically creating an ROI in a changed mode.

In the present disclosure, the user may set a function to automatically create an ROI to be turned on or off through the input unit 150. The user may adjust various methods for creating an ROI through the input unit 150.

For example, in response to a manipulation of the user, the image measurer 500 may only measure the breadth of the object. The upper (first) screen of FIG. 6 briefly depicts that only the breadth of an object image is measured.

If the user makes a mode change later, the image generator 300 may create an image according to the changed diagnosis mode.

The ROI generator 400 creates an ROI in the form of a rectangular box by extending the measured breadth of the object image to a certain extent. However, unlike FIG. 5, since the length of the object image is not measured, the ROI generator 400 may use a predetermined length value in creating the ROI in the form of the rectangular box.

In other words, if only one of the length and breadth of the object image has been measured, the ROI generator 400 may use a default length or default breadth in creating an ROI. The user may adjust the default value through the input unit 150 as well.

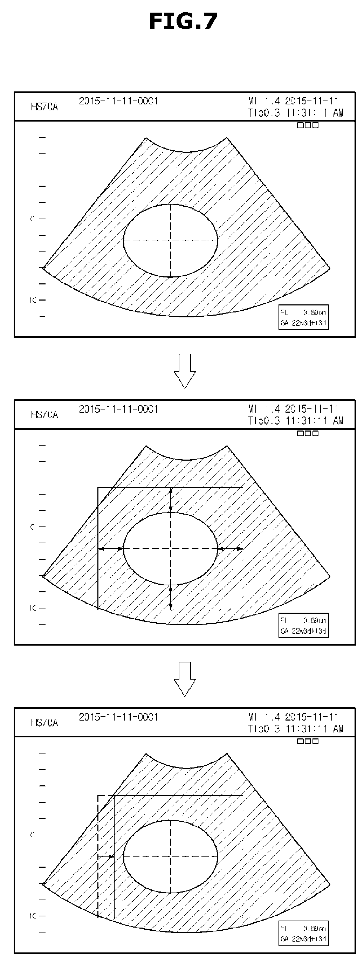

FIG. 7 shows an example of automatically creating an ROI by editing an ROI created beyond the image screen. Processes of creating and displaying an ultrasound image, which overlap what are described above in connection with FIGS. 5 and 6 will not be described herein.

In the upper view (first screen) of FIG. 7, the image measurer 500 measures an object image in the first mode. After this, the ROI generator 400 may create an ROI with predetermined values as shown in the middle view (second screen) of FIG. 7.

That is, the second screen of FIG. 7 corresponds to an image to be created after the user makes a mode change from the first mode to the second mode.

The ROI generator 400 may determine whether the created ROI goes beyond the size of an image in which the ROI is to be displayed, before sending the ROI to the display 160. If the created ROI goes beyond the image as shown in the second screen, the ROI generator 400 may automatically edit the ROI.

Referring to the lower view (third screen) of FIG. 7, the ROI generator 400 may adjust the breadth of the ROI to fit the image in which the ROI is to be displayed. Specifically, if the size of the ROI created by applying the predetermined values goes beyond the image, the ROI generator 400 may edit the ROI by adjusting the size of the ROI without intervention of the user and the resized ROI may be displayed through the display 160.

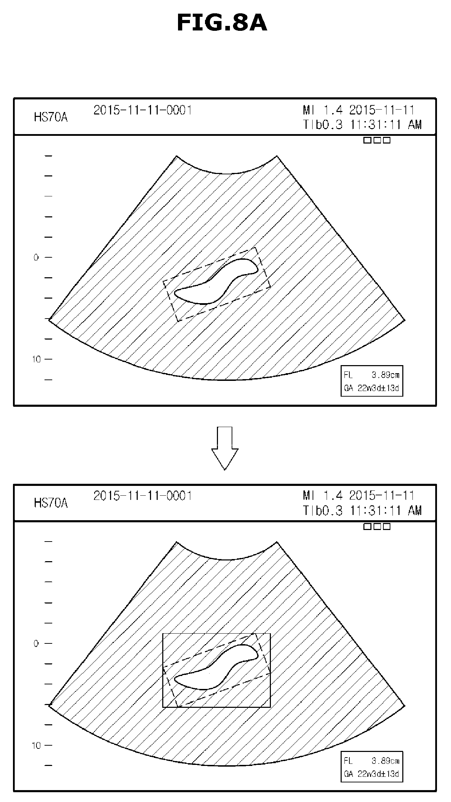

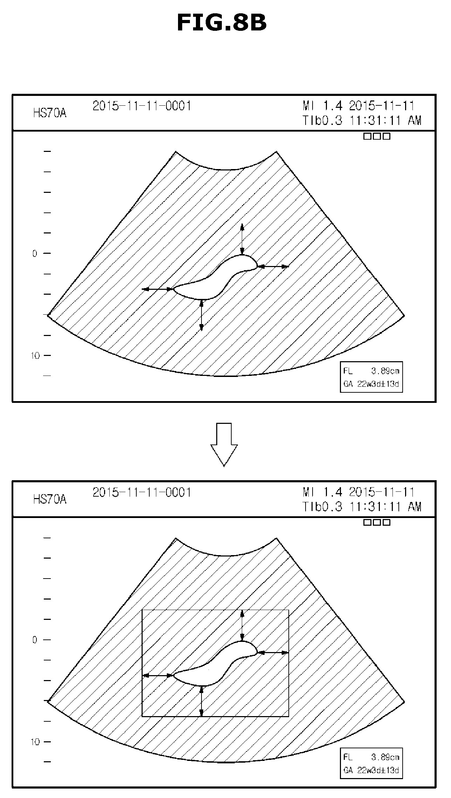

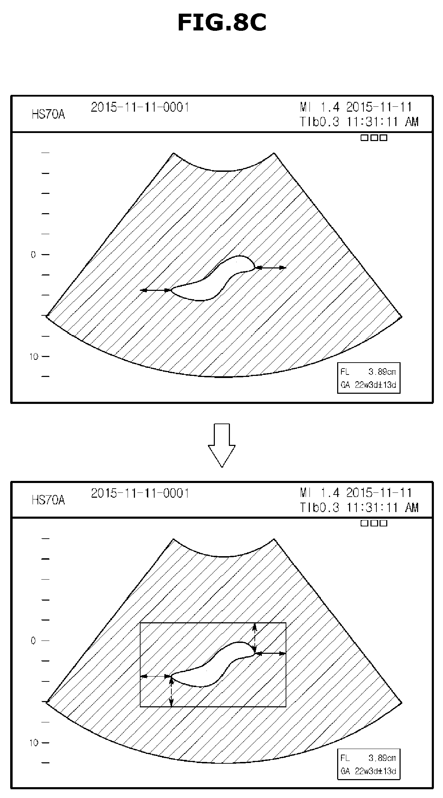

FIGS. 8A, 8B, and 8C are views for explaining methods for measuring a mass, which is diagonally positioned, and creating an ROI.

There may be various object images created by the image generator 300. Also, there may be various methods for the image measurer 500 to measure the object image. In FIGS. 8A, 8B, and 8C, various methods for measuring a diagonal shaped object inclined at an angle and creating an ROI based on the measurements are illustrated.

Referring first to FIG. 8A, the upper (first) image represents a result of measuring an object image. The measured size of the object image is represented by a rectangle inclined at an angle.

When a mode change is made, the ROI generator 400 may create an ROI based on angular points of the measured rectangle. The ROI generator 400 creates the ROI as shown in the middle (second) view of FIG. 8A and sends it to the display 160, and the display 160 may display an image in the changed second mode and the created ROI.

Referring to FIG. 8B, a method for measuring an object of an irregular form is illustrated. The image measurer 500 measures the object displayed on an image in the first mode. Specifically, the image measurer 500 measures the length and breadth of the object based on a point in the object image designated by the user through the input unit 150.

Bidirectional arrows shown in the upper (first) image of FIG. 8b refer to a certain part of the image designated by the user through the input unit 150. The image measurer 500 may measure the length and breadth of the object based on the certain part.

The lower (second) image of FIG. 8b shows an ROI generated by the ROI generator 400 based on the measured size as a mode change is made. Specifically, the ROI generator 400 may automatically create and display an ROI based on the part designated by the user in the first mode, after the mode change is made.

Referring to FIG. 8C, the upper (first) view shows that only the breadth of the object image is measured by the image measurer 500. The lower (second) view shows an image and ROI created as a mode change is made.

Specifically, the ROI generator 400 determines the length of the object image to have a default value based on a part of the object image from which the measured breadth begins, and creates an ROI based on the measured breadth and the default length.

That is, if the user designates a part of the image through the input unit 150 to determine the breadth of the object, the ROI generator 400 may determine the length of an ROI to be created, which starts from a part of the image designated by the user in the second mode and extends as long as a predetermined length, to create an ROI.

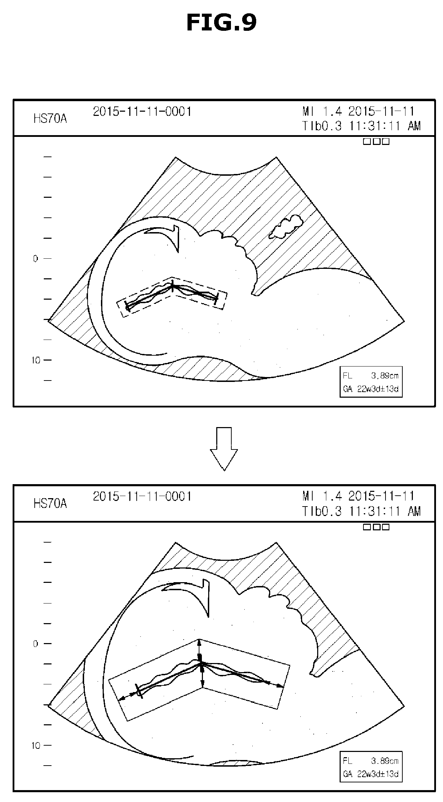

FIGS. 9 to 11 show examples of automatically creating an ROI for various forms of object images. What is overlapped with the description in connection with FIG. 5 will be omitted herein.

An object to be analyzed by the ultrasonic diagnostic apparatus may vary in size and shape. The object image to be displayed may vary accordingly.

Among the images or ROI to be created in many different forms, the upper (first) view of FIG. 9 illustrates an object image in the form of a bending straight line in the first mode.

Referring to the first view of FIG. 9, the image measurer 500 may measure the object image as having the shape of a bending straight line. The shape of a bending straight line refers to a shape in which two straight lines are linked at a certain bending angle.

Specifically, the image measurer 500 may measure the object in the shape of a bending straight line based on a point designated by the user through the input unit 150. For example, the image measurer 500 may measure a distance from the bending point to the user-designated point.

After a mode change is made, the ROI generator 400 may generate an ROI to have a predetermined size based on the bending point on an image under the second mode. Specifically, the ROI generator 400 may generate an ROI in the form of a bending rectangle extending from the user-designated point to a certain extent.

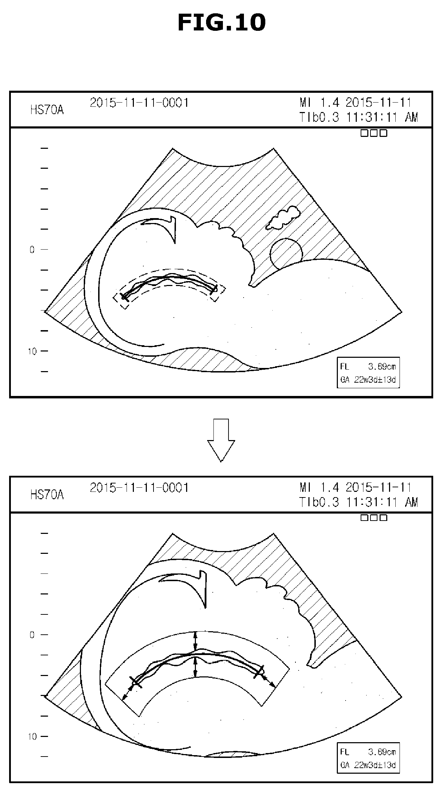

Referring to the upper (first) view of FIG. 10, the image measurer 500 may measure a curved object image according to the Trace measuring method. A curved shape refers to a shape having a curvature.

After a mode change is made, the ROI generator 400 may generate an ROI by extending the traced area of the curved shape to a certain extent on an image under the second mode. Alternatively, the ROI generator 400 may generate a curved ROI by extending from a user-designated trace point to a certain extent.

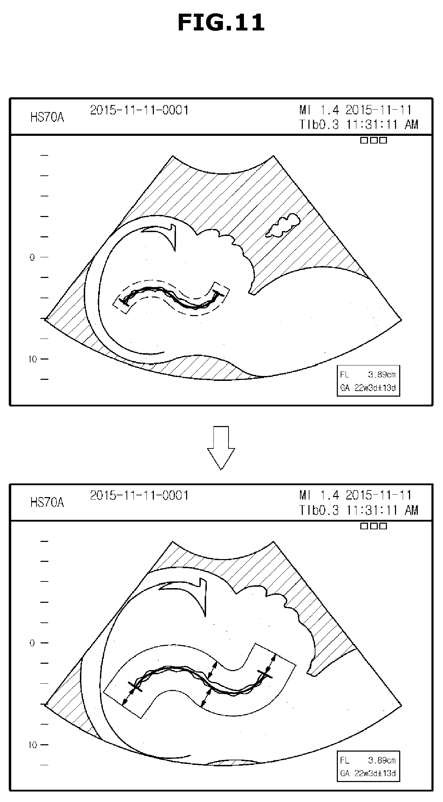

Referring to the upper (first) view of FIG. 11, the image measurer 500 may measure an object image in the shape of a free curve. The shape of a free curve refers to a curve having irregular curvatures. There may be various methods for measuring such a free curved shape, and the user may designate a defined shape through the input unit 150.

After a mode change is made, the ROI generator 400 may automatically create an ROI by modifying measurements of the free curved object to some extent based on the measured shape. The ROI created in this example may be in a free curved shape.

As shown in FIGS. 9 to 11, the ROI generator 400 may create various forms of ROI based on the object image measured in the first mode, as a mode change is made. Many different forms shown in FIGS. 9 to 11 are only by way example, and have no limitations.

The user may also edit the ROI created in the second mode through the input unit 150. In other words, it is possible for the user to re-adjust the created ROI, and there is no limitations.

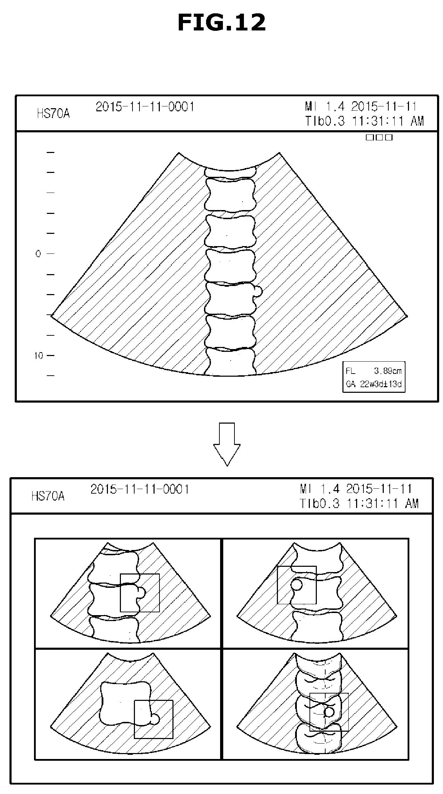

FIG. 12 shows an example of creating an ROI when a 2D mode is changed to a 3D mode.

In the upper (first) screen of FIG. 12, there is an image in a 2D mode in which a spine is simply depicted. In the image, an object such as a mass is formed in the third spinal joint from the bottom.

If the user makes a change from a first mode to a second mode through the input unit 150, the image generator 300 may create an image as shown in the middle (second) view of FIG. 12. The second mode herein refers to a 3D mode.

Specifically, the upper left screen in the second view of FIG. 12 displays an image with the mass viewed from the front. That is, the upper left screen displays an image viewed along the y-axis in a 3D space represented with the xyz axes.

The upper right screen of FIG. 12 displays an image with the mass viewed from the right. That is, the upper right screen displays an image viewed along the x-axis in the 3D space.

The lower left screen of FIG. 12 displays an image with the mass viewed from the top. That is, the lower left screen of FIG. 12 displays an image viewed along the z-axis in the 3D space.

The lower right screen of FIG. 12 simply depicts the first mode image of FIG. 12 into a 3D mode. Specifically, the lower right screen of FIG. 12 displays an image resulting from combination of the previous three images.

The second mode images (in the lower view) of FIG. 12 may each be displayed with an ROI in the form of a rectangular box. Specifically, when a mode change is made, the ROI generator 400 creates a form of a rectangular box expanding from an object image measured by the image measurer 500 in the first mode to a certain extent, and accordingly creates an ROI to be displayed together on the image in the 3D mode.

According to embodiments of an ultrasonic diagnostic apparatus and method for controlling the same, an ROI may be automatically created to fit a changed object image if the user makes a mode change, thereby increasing the user convenience and shortening time required for the user to re-adjust the ROI.

In addition to what are described above, the ROI generator 400 may create the ROI in many different forms, and other embodiments that may be contemplated by an ordinary person in the art may also be included in the present disclosure.

### [Description of the Symbols]

- 100:: MAIN UNIT
- 130:: CABLE
- 140:: MAIL CONNECTOR
- 145:: FEMALE CONNECTOR
- 150:: INPUT UNIT
- 160:: DISPLAY
- 161:: SUB DISPLAY PANEL
- 200:: PROBE
- 300:: IMAGE GENERATOR
- 400:: ROI GENERATOR
- 500:: IMAGE MEASURER

## Claims

1. An ultrasonic diagnostic apparatus comprising:
a probe for irradiating an ultrasound signal to an object and receiving an echo ultrasound signal from the object;
an image generator for creating an image in a first mode based on the echo ultrasound signal received by the probe and creating an image in a second mode related to the first mode;
an image measurer for measuring the object image on the image in the first mode;
a region of interest (ROI) generator for automatically creating an ROI in the second mode based on the measured object image as the first mode is changed to the second mode; and
a display for displaying the image in the first mode, and displaying the image in the second mode with the created ROI as the mode change is made.

2. The ultrasonic diagnostic apparatus of claim 1,
wherein the image in the first mode comprises an image created in B mode and the second mode comprises at least one of power Doppler mode, color Doppler mode, directional power Doppler mode, elasticity mode, and 3D mode.

3. The ultrasonic diagnostic apparatus of claim 1 or 2,
wherein the image generator includes an input unit for receiving a command to control at least one of the image measurer and the ROI generator.

4. The ultrasonic diagnostic apparatus of claim 3,
wherein the image generator is configured to create the image in the second mode related to the image in the first mode under the control of the input unit and the ROI generator is configured to edit the ROI created in the second mode under the control of the input unit.

5. The ultrasonic diagnostic apparatus of claim 3 or 4,
wherein the image measurer is configured to measure a size or volume of the object image in the first mode under the control of the input unit.

6. The ultrasonic diagnostic apparatus of claim 5,
wherein the ROI generator is configured to create the ROI according to setting values input through the input unit based on the size or volume of the object measured by the image measurer.

7. The ultrasonic diagnostic apparatus of claim 6,
wherein the ROI generator is configured to create the ROI according to a setting value of unmeasured length or breadth of the object input through the input unit if the image measurer measures one of the length and breadth of the object.

8. The ultrasonic diagnostic apparatus of claim 6 or 7,
wherein the ROI generator is configured to modify the ROI to fit the image in the second mode, if the ROI goes beyond the image in the second mode.

9. The ultrasonic diagnostic apparatus of claim 6, 7 or 8,
wherein the ROI generator is configured to create an ROI in the second mode based on angular points of a measured area of the object, if the image measurer measures the object while rotating around the object.

10. The ultrasonic diagnostic apparatus of any of claims 6-9,
wherein the ROI generator is configured to create an ROI in the second mode based on a point at which the measured object is bent, if the image measurer measures the object as having the form of a bending straight line in measuring the size of the object.

11. The ultrasonic diagnostic apparatus of any of claims 6-10,
wherein the ROI generator is configured to create an ROI in the second mode based on a measured curvature of a curve, if the image measurer measures the object as having the form of the curve or a free curve in measuring the size of the object.

12. A method for controlling an ultrasonic diagnostic apparatus comprising:
irradiating an ultrasound signal to an object and receiving an echo ultrasound signal from the object;
creating an image according to a mode based on the received echo ultrasound signal;
measuring the object image on the image in a first mode;
creating a region of interest (ROI) in a second mode based on the measured object image as the first mode is changed to the second mode; and
displaying the image in the first mode, and displaying the image in the second mode with the created ROI as the mode change is made.

13. The method of claim 12,
wherein the image in the first mode comprises an image created in B mode and the second mode comprises at least one of power Doppler mode, color Doppler mode, directional power Doppler mode, elasticity mode, and 3D mode.

14. The method of claim 12 or 13,
wherein measuring the object image on the image in a first mode comprises measuring a size or volume of the object image on the image in the first mode and creating an ROI comprises creating the ROI according to setting values based on the measured size or volume of the object.

15. The method of claim 12, 13 or 14, further comprising:
editing the created ROI under the control of a user.
